Europäisches Patentamt

European Patent Office

Office européen des brevets

(11) Publication number: **0 180 269**
**B1**

(12) **EUROPEAN PATENT SPECIFICATION**

(45) Date of publication of patent specification: **27.06.90**

(21) Application number: **85201622.9**

(22) Date of filing: **07.10.85**

(51) Int. Cl.⁵: **B 01 J 23/74, B 01 J 37/02**

(54) **Catalyst preparation.**

(30) Priority: **02.11.84 NL 8403335**

(43) Date of publication of application:
**07.05.86 Bulletin 86/19**

(45) Publication of the grant of the patent:
**27.06.90 Bulletin 90/26**

(84) Designated Contracting States:
**AT BE DE FR GB IT NL SE**

(56) References cited:
**EP-A-0 044 740**
**EP-A-0 104 672**
**FR-A-2 347 097**
**GB-A-2 077 289**
**US-A-2 722 504**
**US-A-4 198 320**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Sudhölter, Ernst Jan Robert TH Twente**
**Afd. Chemietechnologie Lab. voor Org. Chemie**
**P.O. Box 217 NL-7500 AE Enschede (NL)**

(74) Representative: **Aalbers, Onno et al**
**P.O. Box 302**
**NL-2501 CH The Hague (NL)**

EP 0 180 269 B1

Courier Press, Leamington Spa, England.

**Description**

The invention relates to a process for the preparation of catalysts comprising cobalt supported on a silica-containing carrier and also to the use of these catalysts in the preparation of hydrocarbons from mixtures of carbon monoxide with hydrogen.

The preparation of hydrocarbons from a $H_2/CO$ mixture by contacting this mixture at elevated temperature and pressure with a catalyst is known in the literature as the Fischer-Tropsch hydrocarbon synthesis. Catalysts suitable for the purpose are those comprising cobalt supported on a silica-containing carrier. Such catalysts can be prepared by contacting a silica-containing carrier with a cobalt compound in the presence of a liquid, then removing the liquid and calcining and activating the composition obtained. In this mamner of catalyst preparation part of the cobalt reacts with hydroxyl groups occurring at the surface of the silica-containing carrier, thus forming cobalt hydroxysilicates, which compounds will not be reduced in the subsequent catalyst activation. Consequently, part of the cobalt has no activity for the Fischer-Tropsch synthesis and therefore the catalysts show lower activity than one would have expected in reliance upon their cobalt contents. It has now been found that this drawback can be overcome by first treating the silica-containing carrier with a compound chosen from a certain group of silicon compounds, then contacting the carrier thus treated with a cobalt compound in the presence of an organic liquid and finally, after removal of the organic liquid, calcining and activating the composition obtained. Eligible for the present purpose are silicon compounds having the general formula $Si(X)_n(Y)_m(Z)$, wherein X=halogen, alkoxy and/or acyloxy; Y=H and/or a hydrocarbyl group; Z=halogen or a nitrogen-containing or an oxygen-containing group; n=0—3; n+m=3.

The present patent application therefore relates to a process for the preparation of catalysts comprising cobalt on a silica-containing carrier, in which a silica-containing carrier is treated with a silicon compound of the general formula $Si(X)_n(Y)_m(Z)$, in which the thus treated carrier is contacted with a cobalt compound in the presence of an organic liquid, in which the organic liquid is removed and in which the composition obtained is calcined and activated.

Treatment of the carrier with a silicon compound according to the invention can be carried out either in the gaseous phase or in the liquid phase. The preferred treatment time is 1—24 hours and in particular 4—20 hours. The treatment is preferably carried out using a solution of the silicon compound in an organic solvent containing no hydroxyl groups. Suitable solvents are octane, benzene, toluene, xylene, acetonitrile and dimethyl sulfoxide. Preference is given to the use of a hydrocarbon or a mixture of hydrocarbons as the solvent. The treatment of the carrier with a solution of a silicon compound in a solvent is preferably carried out at a temperature of 40—200°C and in particular of 80—150°C. Said treatment can very suitably be carried out by boiling the carrier and the solution of the silicon compound under reflux.

If in the process according to the invention a silicon compound is used in which halogen, alkoxy or acyloxy occurs as group X, preference is given to chlorine, methoxy or ethoxy and acetyloxy, respectively. If a silicon compound is used in which a hydrocarbyl group occurs as group Y, preference is given to methyl and ethyl. If a silicon compound is used in which as the group Z an oxygen-containing group occurs which does not belong to the alkoxy and acyloxy class, or in which a nitrogen-containing group occurs as group Z, preference is given to —O—Si(Y)$_3$ and —NH—Si(Y)$_3$. In the process according to the invention preference is given to the use of silicon compounds of the general formula $Si(X)_2(Y)(Z)$, and in particular those in which the two groups X are similar.

Examples of suitable silicon compounds which are eligible for use in the process according to the invention that may be mentioned are:

$Si(OC_2H_5)_3H$ = triethyloxysilane;
$Si(CH_3)_3Cl$ = trimethylchlorosilane;
$Si(CH_3COO)_3H$ = triacetyloxysilane;
$SiCl_4$ = tetrachlorosilane;
$[(CH_3)_3Si]_2NH$ = di(trimethylsilyl)amine;
$[(CH_3)_3Si]_2O$ = di(trimethylsilyl)oxide.

The catalysts which are prepared according to the invention comprise cobalt supported on a silica-containing carrier. Suitable carriers are inter alia silica, silica-alumina and silica-magnesia. Preference is given to the use of silica as carrier. The quantities of cobalt present on the catalysts may vary within wide ranges. It is preferred to prepare catalysts comprising 3—60 parts by weight of cobalt and in particular 5—50 parts by weight of cobalt per 100 parts by weight of carrier material. The catalysts which are prepared according to the invention preferably comprise one or more promoters. Promoters suitable for the present cobalt catalysts are iron, magnesium, zinc and thorium. It is preferred to prepare catalysts which comprise zirconium, titanium, chromium or ruthenium as promoter. Special preference is given to the use of zirconium as promoter. The preferred quantity of promoter present on the cobalt catalysts is 0.1—5 parts by weight per 100 parts by weight of carrier. The preparation of catalysts according to the invention which, in addition to cobalt, comprise a promoter is preferably carried out by first depositing the cobalt on the carrier, and after calcination of the cobalt-loaded carrier, the promoter. If desired, the cobalt and the promoter may be deposited on the carrier simultaneously.

The deposition of the cobalt is carried out by contacting the carrier which has been treated with the silicon compound with a cobalt compound in the presence of an organic liquid. For this purpose use is preferably made of a solution of a cobalt compound in an organic solvent. Very suitable solvents for the purpose are methanol, ethanol and glycol. If the deposition of the cobalt onto the carrier using a solution of a cobalt compound in an organic solvent is carried out in the conventional manner by contacting the carrier for a considerable period of time at room temperature with a relatively dilute cobalt solution, this leads to catalysts in which the cobalt is distributed homogeneously over the carrier, viz. at every point in the catalyst particle the cobalt concentration is virtually the same. A recent investigation into the use of catalysts comprising cobalt supported on a carrier for the preparation of hydrocarbons from $H_2$/CO mixtures has shown that catalysts in which the cobalt distribution over the carrier material is inhomogeneous in a specific manner, show higher $C_5^+$ selectivities than similar catalysts in which the cobalt is distributed over the carrier homogeneously. It has been found that the desired inhomogeneous cobalt distribution over the carrier is attained by submersing the carrier once or several times in a solution of a cobalt compound in an organic solvent, removing the liquid from the composition after each submersion, and seeing to it that during each submersion the following relation between viscosity measured in cS at 60°C (1 cS=$10^{-6}$ m²/S) and temperature (T in °K) of the solution, and the submersion time (t in s) is satisfied:

$$\frac{\log v}{t \times T} \times 10^4 \geq 1.$$

In the catalyst preparation according to the invention it is preferred to make use of this finding.

The present patent application also relates to the use of the catalysts prepared according to the invention for the conversion of a $H_2$/CO mixture into hydrocarbons. Preparatory to becoming eligible for this purpose, the cobalt catalysts should be activated. This activation can suitably be carried out by contacting the catalysts at a temperature between 200 and 350°C with hydrogen or a hydrogen containing gas.

The conversion of the $H_2$/CO mixture into hydrocarbons by using a catalyst according to the invention is preferably carried out at a temperature of 125—350°C and in particular of 175—275°C and a pressure of 5—100 bar and in particular of 10—75 bar. Further, the conversion is preferably carried out by contacting the $H_2$/CO mixture with a catalyst which is present in the form of a fixed bed.

$H_2$/CO mixtures which are eligible to be converted into hydrocarbons according to the invention can very suitably be obtained by steam reforming or partial oxidation starting from light hydrocarbons, such as natural gas.

The $H_2$/CO mixture which is converted into hydrocarbons according to the invention preferably has a $H_2$/CO molar ratio higher than 1.5. If the feed has a $H_2$/CO molar ratio lower than 1.5, it is preferred to increase the latter to a value lying between 1.5 and 2.5 and in particular between 1.75 and 2.25, before contacting the feed with the cobalt catalyst. The $H_2$/CO molar ratio of hydrogen-poor $H_2$/CO mixtures can be increased by, inter alia, addition of hydrogen, removal of carbon monoxide, mixing with a hydrogen-rich $H_2$/CO mixture or by subjecting the hydrogen-poor $H_2$/CO mixture to the CO-shift reaction.

The conversion of the $H_2$/CO mixture according to the invention can be used as an independent process in which unconverted synthesis gas can be recirculated, if desired. Further, the conversion of the $H_2$/CO mixture according to the invention can very suitably be used as the first step in a two-step process for the preparation of middle distillates from $H_2$/CO mixtures. For it has been found that catalysts containing silica or silica-alumina as carrier and cobalt together with zirconium, titanium, chromium and/or ruthenium as catalytically active metals, which catalysts have been prepared by depositing the metals concerned on the carrier material by impregnation and/or kneading, yield a product substantially consisting of unbranched paraffins whose high-boiling part can be converted in high yield into middle distillates by subjecting it to a hydrocracking treatment.

Although in the preparation of middle distillates from the product obtained over the cobalt catalyst the part of the product whose initial boiling point lies above the final boiling point of the heaviest middle distillate desired as end product will do as feed for the hydrocracking, the total $C_5^+$ fraction of the product prepared over the cobalt catalyst may also be used for the purpose, if desired.

The hydrocracking is carried out by contacting the fraction to be treated at elevated temperature and pressure and in the presence of hydrogen qith a catalyst containing one or more Group VIII noble metals on a carrier. The hydrocracking catalyst used by preference is a catalyst comprising 0.1—2 %w of platinum or palladium supported on silica-alumina as carrier. The hydrocracking treatment is preferably carried out at a temperature of 250—350°C and a pressure of 10—75 bar.

The invention is now illustrated with the aid of the following example.

Example
Catalyst preparation
Nine Co/SiO$_2$ catalysts (Catalysts 1—9), some of which had and some of which had not been promoted with Zr or Ru, were prepared as follows starting from a globular silica carrier which previously had been calcined at 500°C and then exposed to the air for 24 hours (hereinafter referred to as Silica A). The starting material used for the preparation of Catalysts 1—3 was Silica A as such. For the preparation of Catalysts 4—6, a Silica B was used, and for the preparation of Catalysts 7—9, Silica C, the latter two silicas having been prepared from Silica A by treatment with a silicon compound.

Silica B

This silica was prepared by boiling under reflux for 12 hours a mixture of 20 g of Silica A, 10 g of thiethoxysilane and 200 ml of toluene. The Silica B obtained was filtered off, washed three times with methanol and dried in vacuo for 16 hours at 80°C.

Silica C

This silica was prepared in substantially the same manner as Silica B, the difference being that now, instead of 20 g of triethoxysilane, 8 g of di(trimethylsilyl)amine was used.

Catalysts 1, 4 and 7

These catalysts, which per 100 parts by weight of silica comprised 25 parts by weight of cobalt, were prepared by impregnation of the silica carriers concerned with a solution of cobalt nitrate in ethanol, followed by drying and calcination of the compositions at 500°C.

Catalysts 2, 5 and 8

These catalysts, which per 100 parts by weight of silica comprised 25 parts by weight of cobalt and 0.9 parts by weight of zirconium, were prepared by impregnating the silica carriers concerned with a solution of cobalt nitrate in ethanol and, after drying and calcination at 500°C, impregnating the cobalt-loaded carriers with a solution of zirconium nitrate in water, followed by drying and calcination at 500°C.

Catalysts 3, 6 and 9

These catalysts, which per 100 parts by weight of silica comprised 25 parts by weight of cobalt and 0.5 parts by weight of ruthenium, were prepared by impregnating the silica carriers concerned with a solution comprising cobalt nitrate and ruthenium chloride in ethanol, followed by drying and calcination of the compositions at 500°C.

In the preparation of Catalysts 1—9 the quantity of liquid used in each impregnation step was of such a volume as to correspond substantially with the pore volume of the carrier.

Catalysts testing

Catalysts 1—9 were used in nine experiments (Experiments 1—9) in the preparation of hydrocarbons from a mixture of carbon monoxide and hydrogen having a $H_2/CO$ molar ratio 2. The experiments were carried out at a pressure of 20 bar, a temperature of 220°C and a space velocity of 600 $N1.1^{-1}.h^{-1}$ in a reactor containing a solid catalyst bed. Preceding the testing the catalysts were activated by treatment with hydrogen at 250°C. The results of Experiments 1—9 are given in the table.

Of the catalysts mentioned in the table, only Catalysts 4—9 are catalysts according to the invention. In the preparation of these catalysts the cobalt was deposited on a silica carrier which had previously been treated with a silicon compound. Catalysts 1—3, where the cobalt was deposited on a non-pretreated carrier, fall outside the scope of the invention. They have been included in the patent application for comparison.

Of the experiments mentioned in the table, only Experiments 4—9 are experiments according to the invention. In these experiments, which were carried out by using catalysts prepared according to the invention, very high activities were observed. Experiments 1—3 fall outside the scope of the invention. In these experiments, which were carried out by using catalysts which had not been prepared according to the invention, considerably lower activities were attained.

4

## TABLE

| Experiment No. | Catalyst No. | Catalyst composition parts by weight | $H_2+CO$ conversion, %vol |
|---|---|---|---|
| 1 | 1 | 25 Co/100 $SiO_2$ (A) | 55 |
| 2 | 2 | 25 Co/0.9 Zr/100 $SiO_2$ (A) | 60 |
| 3 | 3 | 25 Co/0.5 Ru/100 $SiO_2$ (A) | 63 |
| 4 | 4 | 25 Co/100 $SiO_2$ (B) | 81 |
| 5 | 5 | 25 Co/0.9 Zr/100 $SiO_2$ (B) | 86 |
| 6 | 6 | 25 Co/0.5 Ru/100 $SiO_2$ (B) | 94 |
| 7 | 7 | 25 Co/100 $SiO_2$ (C) | 75 |
| 8 | 8 | 25 Co/0.9 Zr/100 $SiO_2$ (C) | 81 |
| 9 | 9 | 25 Co/0.5 Ru/100 $SiO_2$ (C) | 90 |

## Claims

1. A process for the preparation of catalysts comprising cobalt supported on a silica-containing carrier, characterized in that a silica-containing carrier is treated with a silicon compound of the general formula $Si(X)_n(Y)_m(Z)$, wherein X=halogen, alkoxy and/or acyloxy; Y=H and/or a hydrocarbyl group; Z=halogen or a nitrogen-containing or an oxygen-containing group; n=0–3; n+m=3, that the carrier thus treated is contacted with a cobalt compound in the presence of an organic liquid, that the organic liquid is removed and that the composition obtained is calcined and activated.

2. A process as claimed in Claim 1, characterized in that the treatment time is 1—24 hours.

3. A process as claimed in Claim 2, characterized in that the treatment time is 4—20 hours.

4. A process as claimed in any one of Claims 1—3, characterized in that the treatment is carried out using a solution of a silicon compound in an organic solvent which contains no hydroxy moieties.

5. A process as claimed in Claim 4, characterized in that the solvent used is a hydrocarbon or a mixture of hydrocarbons.

6. A process as claimed in Claim 4 or 5, characterized in that the treatment is carried out at a temperature of 40—200°C.

7. A process as claimed in Claim 6, characterized in that the treatment is carried out at a temperature of 80—150°C.

8. A process as claimed in any one of Claims 4—7, characterized in that the treatment is carried out by boiling the carrier with the solution of the silicon compound under reflux.

9. A process as claimed in any one of Claims 1—8, characterized in that a silicon compound is used in which the occurring group X is halogen, alkoxy or acyloxy, which group is present as chlorine, methoxy or ethoxy, or acetyloxy, respectively.

10. A process as claimed in any one of Claims 1—9, characterized in that a silicon compound is used in which the occurring group Y is a hydrocarbyl group, which group is present as methyl or ethyl.

11. A process as claimed in any one of Claims 1—10, characterized in that a silicon compound is used in which the occurring group Z is an oxygen-containing group which does not belong to the alkoxy and acyloxy class, or in which the occurring group Z is a nitrogen-containing group, which group is present as $-O-Si(Y)_3$ or $-NH-Si(Y)_3$, respectively.

12. A process as claimed in any one of Claims 1—10, characterized that use is made of a silicon compound of the general formula $Si(X)_2(Y)(Z)$.

13. A process as claimed in any one of Claims 1—12, characterized in that per 100 parts by weight of carrier the catalyst comprises 5—50 parts by weight of cobalt and 0.1—5 parts by weight of promoter.

14. A process as claimed in any one of Claims 1—13, characterized in that the catalyst comprises one or more promoters chosen from zirconium, titanium, chromium and ruthenium.

15. A process as claimed in any one of Claims 1—14, characterized in that the carrier is submersed in a cobalt containing solution once or several times, that after each submersion the liquid is removed from the composition, and that during each submersion the following relation between viscosity measured at 60°C in cS (1 cS=$10^{-6}$m²/s) and temperature (T in °K) of the solution and the submersion time (t in s) is satisfied:

$$\frac{\log v}{t \times T} \times 10^4 \geq 1.$$

16. Catalysts which comprise cobalt supported on a silica-containing carrier and which have been prepared by a process as described in Claim 1.

17. A process for the preparation of hydrocarbons by catalytic reaction of carbon monoxide with hydrogen, characterized in that a $H_2$/CO mixture is contacted at elevated temperature and pressure with a catalyst as claimed in Claim 16.

18. A process as claimed in Claim 17, characterized in that it is used as the first step in a two-step process for the preparation of middle distillates from $H_2$/CO mixtures, that in the first step use is made of a catalyst which comprises silica or silica-alumina as carrier and cobalt together with zirconium, titanium, chromium and/or ruthenium as catalytically active metals, which catalyst has been prepared by depositing the metals concerned on the carrier material by impregnation and/or kneading, and that of the product prepared in the first step at least the part whose initial boiling point lies above the final boiling point of the heaviest middle distillate desired as end product is subjected in the second step to a hydrocracking treatment by contacting it at elevated temperature and pressure with a catalyst comprising one or more noble metals from Group VIII supported on a carrier.

**Patentansprüche**

1. Verfahren zur Herstellung von Katalysatoren, die Kobalt, aufgebracht auf einem Siliziumdioxidenthaltenden Träger, umfassen, dadurch gekennzeichnet, daß ein Siliciumdioxid enthaltender Träger mit einer Siliziumverbindung der allgemeinen Formel Si(X)$_n$(Y)$_m$(Z), worin

X=Halogen, Alkoxy und/oder Acyloxy;
Y=H und/oder eine Hydrocarylgruppe;
Z=Halogen oder ein Stickstoff-enthaltende oder eine Sauerstoffenthaltende Gruppe;
n=0—3;
n+m=3 ist,

behandelt wird, daß der solcherart behandelte Träger mit einer Kobaltverbindung in Gegenwart einer organischen Flüssigkeit in Berührung gebracht wird, daß die organische Flüssigkeit abgetrennt wird und daß die erhaltene Zusammensetzung calciniert und aktiviert wird.

2. Verfahren nach Anspruch 1, dadurch gekennzeichnet, daß die Behandlungsdauer 1 bis 24 Stunden beträgt.

3. Verfahren nach Anspruch 2, dadurch gekennzeichnet, daß die Behandlungsdauer 4 bis 20 Stunden beträgt.

4. Verfahren nach einem der Ansprüche 1 bis 3, dadurch gekennzeichnet, daß die Behandlung unter Verwendung einer Lösung einer Siliziumverbindung in einem organischen Lösungsmittel, das keine Hydroxyreste enthält, ausgeführt wird.

5. Verfahren nach Anspruch 4, dadurch gekennzeichnet, daß das verwendete Lösungsmittel ein Kohlenwasserstoff oder ein Gemisch von Kohlenwasserstoffen ist.

6. Verfahren nach Anspruch 4 oder 5, dadurch gekennzeichnet, daß die Behandlung bei einer Temperatur von 40 bis 200°C ausgeführt wird.

7. Verfahren nach Anspruch 6, dadurch gekennzeichnet, daß die Behandlung bei einer Temperatur von 80 bis 150°C ausgeführt wird.

8. Verfahren nach einem der Ansprüche 4 bis 7, dadurch gekennzeichnet, daß die Behandlung durch Rückflußsieden des Trägers mit der Lösung der Siliziumverbindung ausgeführt wird.

9. Verfahren nach einem der Ansprüche 1 bis 8, dadurch gekennzeichnet, daß eine Siliziumverbindung verwendet wird, worin die auftretende Gruppe X Halogen, Alkoxy oder Acyloxy ist, welche Gruppe als Chlor, Methoxy oder Ethoxy bzw. Acetyloxy zugegen ist.

10. Verfahrfen nach einem der Ansprüche 1 bis 9, dadurch gekennzeichnet, daß eine Siliziumverbindung verwendet wird, worin die vorliegende Gruppe Y eine Hydrocarbylgruppe ist, welche Gruppe als Methyl oder Ethyl zugegen ist.

11. Verfahren nach einem der Ansprüche 1 bis 10, dadurch gekennzeichnet, daß eine Siliziumverbindung verwendet wird, worin die vorliegende Gruppe Z eine Sauerstoff-enthaltende Gruppe ist, die nicht zu der Alkoxy- oder Acyloxyklasse gehört, oder worin die vorliegende Gruppe Z eine Stickstoffenthaltende Gruppe ist, welche Gruppe als —O—Si(Y)$_3$ bzw. —NH—Si(Y)$_3$ zugegen ist.

12. Verfahren nach einem der Ansprüche 1 bis 10, dadurch gekennzeichnet, daß die einer Siliziumverbindung der allgemeinen Formel Si(X)$_2$(Y)(Z) Gebrauch gemacht wird.

13. Verfahren nach einem der Ansprüche 1 bis 12, dadurch gekennzeichnet, daß der Katalysator je 100 Gewichtsteile Träger 5 bis 50 Gewichtsteile Kobalt und 0,1 bis 5 Gewichtsteile Promotor aufweist.

14. Verfahren nach einem der Ansprüche 1 bis 13, dadurch gekennzeichnet, daß der Katalysator einen oder mehrere Promotoren, ausgewählt unter Zirkon, Titan, Chrom und Ruthenium, umfaßt.

15. Verfahren nach einem der Ansprüche 1 bis 14, dadurch gekennzeichnet, daß der Träger in eine Kobalt-enthaltende Lösung einmal oder mehrmals untergetaucht wird, daß nach jedem Untertauchen die Flüssigkeit von der Zusammensetzung abgetrennt wird und daß während jedes Tauchvorganges die nachstehende Beziehung zwischen der bei 60°C gemessenen Viskosität in cS (1 cS=10⁻⁶ m²/s) und der Temperatur (T in °K) der Lösung und der Tauchzeit (t in s) erfüllt wird:

$$\frac{\log v}{t \times T} \times 10^4 \geqslant 1.$$

16. Katalysatoren, die Kobalt, aufgebracht auf einem Siliziumdioxid enthaltenden Träger, umfassen und die nach einem Verfahren, wie in Anspruch 1 beschrieben, hergestellt worden sind.

17. Verfahren zur Herstellung von Kohlenwasserstoffen durch katalytische Umsetzung von Kohlenmonoxid mit Wasserstoff, dadurch gekennzeichnet, daß ein $H_2$/CO-Gemisch bei erhöhter Temperatur und bei erhöhtem Druck mit einem Katalysator, wie in Anspruch 16 beansprucht, in Berührung gebracht wird.

18. Verfahren nach Anspruch 17, dadurch gekennzeichnet, daß es als die erste Stufe in einem Zweitstufenverfahren zur Herstellung von Mitteldestillaten aus $H_2$/CO-Gemischen angewendet wird, daß in der ersten Stufe von einem Katalysator Gebrauch gemacht wird, der Siliziumdioxid oder Siliziumdioxid-Aluminiumoxid als Träger und Kobalt zusammen mit Zirkon, Titan, Chrom und/oder Ruthenium als katalytisch aktive Metalle umfaßt, welcher Katalysator durch Ablagern der entsprechenden Metalle auf dem Trägermaterial durch Imprägnieren und/oder Kneten hergestellt worden ist, und daß von dem in der ersten Stufe hergestellten Produkt zumindest jener Teil, dessen Anfangssiedepunkt über dem Endsiedepunkt des als Endprodukt gewünschten schwersten Mitteldestillates liegt, in der zweiten Stufe einer Hydrocrackbehandlung unterworfen wird, indem er bei erhöhter Temperatur und bei erhöhtem Druck mit einem Katalysator in Berührung gebracht wird, der ein oder mehrere Edelmetalle aus der Gruppe VIII, aufgebracht auf einem Träger, umfaßt.

**Revendications**

1. Un procédé pour la préparation de catalyseurs comprenant du cobalt déposé sur un support contenant de la silice, caractérisé en ce qu'un support contenant de la silice est traité par un composé du silicium de la formule générale $Si(X)_n(Y)_m(Z)$, où X=halogène, alcoxy et/ou acyloxy; Y=H et/ou un groupe hydrocarbyl; Z=un halogène ou un groupe contenant de l'azote ou contenant de l'oxygène; n=0—3; n+m=3, que le support ainsi traité est mis en contact avec un composé du cobalt en présence d'un liquide organique, que le liquide organique est éliminé et que la composition obtenue est calcinée et activée.

2. Un procédé selon la revendication 1, caractérisé en ce que la durée du traitement est de 1—24 heures.

3. Un procédé selon la revendication 2, caractérisé en ce que la durée du traitement est de 4—20 heures.

4. Un procédé selon l'une quelconque des revendications 1—3, caractérisé en ce que le traitement est effectué en utilisant une solution d'un composé du silicium dans un solvant organique qui ne contient pas de portions hydroxy.

5. Un procédé selon la revendication 4, caractérisé en ce que le solvant utilisé est un hydrocarbure ou un mélange d'hydrocarbures.

6. Un procédé selon la revendication 4 ou 5, caractérisé en ce que le traitement est effectué à une température de 40—200°C.

7. Un procédé selon la revendication 6, caractérisé en ce que le traitement est effectué à une température de 80—150°C.

8. Un procédé selon l'une quelconque des revendications 4—7, caractérisé en ce qu'on effectue le traitement en faisant bouillir au reflux le support avec la solution du composé du silicium.

9. Un procédé selon l'une quelconque des revendications 1—8, caractérisé en ce qu'on utilise un composé du silcium dans lequel le groupe X présent est un halogène ou un groupe alcoxy ou acyloxy, ce groupe étant présent sous la forme de chlore, de groupe méthoxy ou éthoxy ou de groupe acétyloxy, respectivement.

10. Un procédé selon l'une quelconque des revendications 1—9, caractérisé en ce qu'on utilise un composé du silcium dans lequel le groupe Y présent est un groupe hydrocarbyle, ce groupe étant présent sous la forme d'un groupe méthyle ou éthyle.

11. Un procédé selon l'une quelconque des revendications 1—10, caractérisé en ce qu'on utilise un composé du silicium dans lequel le groupe Z présent est un groupe contenant de l'oxygène qui n'appartient pas à la classe des groupes alcoxy et acyloxy, ou dans lequel le groupe Z présent est une groupe contenant de l'azote, ce groupe étant présent sous la forme de $-O-Si(Y)_3$ ou $-NH-Si(Y)_3$, respectivement.

12. Un procédé selon l'une quelconque des revendications 1—10, caractérisé en ce qu'on utilise un composé du silicium de la formule générale $Si(X)_2(Y)(Z)$.

13. Un procédé selon l'une quelconque des revendications 1—12, caractérisé en ce que pour 100 parties en poids de support le catalyseur comprend 5—50 parties en poids de cobalt et 0,1—5 parties en poids de promoteur.

14. Un procédé selon l'une quelconque des revendications 1—13, caractérisé en ce que le catalyseur comprend un ou plusieurs promoteurs choisis parmi le zirconium, le titane, le chrome et le ruthénium.

15. Un procédé selon l'une quelconque des revendications 1—14, caractérisé en ce que le support est plongé une ou plusieurs fois dans une solution contenant du cobalt, qu'après chaque immersion le liquide est éliminé de la composition, et que durant chaque immersion, la relation suivante entre la viscosité mesurée à 60°C en cSt (1 cSt=$10^{-6}$ m²/s), la température (T en °K) de la solution et la durée de l'immersion (t en secondes) est satisfaite:

$$\frac{\log v}{t \times T} \times 10^4 \geqslant 1.$$

16. Des catalyseurs qui comprennent du cobalt déposé sur un support contenant de la silice et qui ont été préparés par un procédé selon la revendication 1.

17. Un procédé pour la préparation d'hydrocarbures par réaction catalytique d'oxyde de carbone avec l'hydrogène, caractérisé en ce qu'un mélange $H_2$/CO est mis en contact à température et pression élevées avec un catalyseur selon la revendication 16.

18. Un procédé selon la revendication 17, caractérisé en ce qu'il est utilisé comme première étape dans un procédé à deux étapes pour la préparation de distillats moyens à partir de mélanges $H_2$/CO, que dans la première étape on utilise un catalyseur qui comprend de la silice ou de la silice-alumine comme support et du cobalt en même temps que du zirconium, du titane, du chrome et/ou du ruthénium comme métaux catalytiquement actifs, ce catalyseur ayant été préparé en déposant les métaux concernés sur la matière de support par imprégnation et/ou malaxage et que, du produit préparé dans la première étape, au moins la partie dont le point initial d'ébullition se trouve au-dessus du point final d'ébullition du distillat moyen le plus lourd désiré comme produit final est soumise dans la deuxième étape à un traitement d'hydrocraquage par mise en contact à température et pression élevées avec un catalyseur comprenant un ou plusieurs métaux nobles du groupe VIII déposés sur un support.